# EUROPÄISCHE PATENTSCHRIFT

(11) **EP 0 699 038 B1**
(45) Veröffentlichungstag und Bekanntmachung des Hinweises auf die Patenterteilung: **03.03.1999**
(21) Anmeldenummer: 94900045.9
(22) Anmeldetag: 15.11.1993
(51) Int. Cl.: A45D 19/00, A45D 20/20, A61N 5/06

(54) **VERFAHREN ZUR EINWIRKUNG AUF HAARE ZU KOSMETISCHEN ZWECKEN**
COSMETIC HAIR-TREATMENT METHOD
PROCEDE DE TRAITEMENT COSMETIQUE DES CHEVEUX

(30) Priorität: 13.11.1992 DE 4238456
(43) Veröffentlichungstag der Anmeldung: 06.03.1996
(73) Patentinhaber: NWL LASER TECHNOLOGIE GMBH, D-90542 Eckental (DE)
(72) Erfinder: WEIMEL, Erich, D-91220 Schnaittach (DE); HOFMANN, Christoph, D-91611 Lehrberg (DE); WARNKE, Ulrich, D-66133 Saarbrücken/Scheidt (DE)
(74) Vertreter: Münich, Wilhelm, Dr.
(86) Internationale Anmeldenummer: DE9301090
(87) Internationale Veröffentlichungsnummer: WO9410874

(56) Entgegenhaltungen:
- WO-A-91/06279
- DE-U- 9 102 407
- GB-A- 617 491
- GB-A- 1 128 647
- US-A- 4 792 341

## Beschreibung

### Technisches Gebiet

Die Erfindung bezieht sich auf ein verfahren zur Einwirkung auf Haare und insbesondere das menschliche Kopfhaar zu kosmetischen Zwecken.

### Stand der Technik

Ein verfahren und eine Vorrichtung gemäß dem Oberbegriff der Patentansprüche 1 bzw. 2 ist aus der US-A-4 792 341 bekannt. Gemäß dieser Druckschrift wird das menschliche Kopfhaar dadurch gebleicht, daß das Haar Licht mit einer Wellenlänge von 694,3 nm ausgesetzt wird. Ein Hinweis darauf, daß durch die Verwendung von Laserlicht der Färbevorgang unterstützt wird, ist dieser Druckschrift nicht zu entnehmen: Beim Bleichen erfolgt nämlich eine Oxydation, während das Färben im wesentlichen auf der Farbstoffeinlagerung in die Haaroberfläche beruht. Insbesondere erfolgt beim Bleichen eine oxidative Zerstörung der Melamin-Pigmente im Haar, während die Färbewirkung auf die Affinität der eingesetzten Farbstoffe zum Haar-Theratin beruht.

Die Erfindung geht nun von folgender Erkenntnis aus:

Haare haben die Tertiärstruktur von Proteinen. Dabei bilden sich eine Reihe von Bindungen zwischen den fibrillären Proteinen, den Mikrofibrillen und den Makrofibrillen aus.

Durch eine gezielte Beeinflussung der Proteinstrukuren mit Licht geeigneter Wellenlänge und Intensität können diese Bindungen beeinflußt werden.

### Darstellung der Erfindung

Erfindungsgemäß wird deshalb gemäß Anspruch 1 das Haar Licht mit einer Intensität und Wellenlänge ausgesetzt, die derart gewählt sind, daß die Proteinstruktur entsprechend der gewünschten kosmetischen Maßnahme beeinflußt wird.

Hierzu wird wenigstens eine Laser-Lichtquelle mit einer geeigneten Wellenlänge verwendet, da sich überraschenderweise herausgestellt hat, daß die Einwirkung auf das Haar zu kosmetischen Zwecken besonders effizient mit kohärentem Licht erfolgt. Unter bestimmten Umständen können jedoch auch nichtkohärentes Licht und damit andere Lichtquellen als Laser verwendet werden.

Erfindungsgemäß ist nämlich erkannt worden, daß sich durch Einstrahlung von Licht mit geeigneter Wellenlänge die Struktur der α-Helices der einzelnen Haar-Makrofibrillen gezielt derart beeinflussen läßt, daß insbesondere die Farben, die mit der chemischen Färbung des Kopfhaares erzielt werden, brillianter und kräftiger als ohne zusätzliche Einstrahlung von Licht erscheinen:

Insbesondere wird zur Unterstützung der chemischen Färbung des Kopfhaars Licht mit einer Wellenlänge zwischen ca 600 nm und 1200 nm eingesetzt, so daß sich eine Enzym-Koordinaten-Änderung bzw. eine Änderung der Redox-Potentiale ergibt.

Bei dem erfindungsgemäßen Verfahren zur färbenden Einwirkung auf Haare wird zunächst in üblicher Weise der Färbevorgang vorgenommen. Anschließend wird das Haar Licht mit einer Intensität und einer Wellenlänge von bevorzugt etwa 780 nm ausgesetzt. Hierdurch wird das Färbeergebnis verbessert, d.h. die Farben wirken brillianter als ohne Lichteinwirkung und es weniger Farbauftrag als bei herkömmlicher Färbung erforderlich.

In den Ansprüchen 2 folgende ist eine Vorrichtung zur Durchführung des erfindungsgemäßen Verfahrens beschrieben.

Da eine Realisierung einer entsprechenden Vorrichtung aufgrund der folgenden Beschreibung für einen Durchschnittsfachmann ohne weiteres möglich ist, wird auf die Darstellung einer bevorzugten Ausführungsform anhand einer Zeichnung verzichtet und diese Ausführungsform lediglich beschrieben:

Erfindungsgemäß weist die Vorrichtung eine Haube auf, deren Innenkontur so ausgebildet ist, daß sie den Haar-Bereich eines menschlichen Kopfes umgibt. In die Haube ist eine Reihe von Laserdioden derart eingesetzt, daß sie von der Innenfläche der Haube Licht in Richtung auf den Haar-Bereich emittieren. Durch diese Ausbildung ist eine einfache großflächige Bestrahlung des Kopfhaares ohne Beeinträchtigung der Umwelt möglich. Vor allem aber erlaubt die Anordnung der Laserdioden in einer geschlossenen Haube die Einstufung einer erfindungsgemäßen Vorrichtung in eine niedrige Laserschutzklasse. Die Laserdioden können dabei insbesondere Licht im Bereich von etwa 780 nm emittieren, da für diesen Spektralbereich genügend leistungsstarke und kostengünstige Laserdioden verfügbar sind.

Die kosmetische Erfolg kann dadurch unterstützt werden, daß zusätzliche Lichtquellen vorgesehen sind, die Licht über einen größeren Wellenlängenbereich emittieren.

Durch die Ausbildung gemäß Anspruch 5, gemäß der in der Haube wenigstens eine Photodiode vorgesehen ist, die mit reflektiertem und/oder von wenigstens einer Laserdiode emittiertem Licht beaufschlagt wird, und deren Ausgangssignal an einer Steuereinheit für die Laserdioden zur Regelung der emittierten Lichtleistung angelegt ist, kann eine zu hohe oder zu geringe Einstrahlung vermieden werden. Die Steuereinheit wird dabei bevorzugt beabstandet von der Haube angeordnet und ist mit dieser über ein Kabel verbunden.

Im Anspruch 6 ist eine Weiterbildung gekennzeichnet, bei der die Haube an einem Ständer befestigt ist, und bei der an der Innenfläche der Haube federartige Abstandshalter vorgesehen sind, die einen Mindestabstand zwischen Innenfläche der Haube und Kopf sicherstellen. Hierdurch ist ein korrekter Abstand zwischen Lichtquellen und Kopf sichergestellt, so daß man ein gleichbleibendes Einwirkungsergebnis erhält.

## Patentansprüche

1. Verfahren zur kosmetischen Einwirkung auf Haare und insbesondere auf das menschliche Kopfhaar, bei dem das Haar Licht und einer Wellenlänge zwischen ca. 600 und 1200 nm ausgesetzt wird,
dadurch **gekennzeichnet**, daß zur Unterstützung der chemischen Färbung wenigstens eine Laserdiode mit einer Wellenlänge und einer Intensität verwendet wird, die so gewählt sind, daß durch die Laserstrahlung die Struktur der α-Helices der einzelnen Haar-Makrofibrillen durch Einwirkung auf die Proteinstruktur der Haare durch eine Enzym-Koordinaten-Änderung bzw. eine Änderung der Redox-Potentiale effizient beeinflußt wird.

2. Vorrichtung zur kosmetischen Einwirkung auf Haare und insbesondere auf das menschliche Kopfhaar, mit einer Lichtquelle, die das Haar mit Licht mit einer Wellenlänge zwischen ca. 600 und 1200 nm bestrahlt,
dadurch **gekennzeichnet**, daß eine Haube vorgesehen ist, deren Innenkontur so ausgebildet ist, daß sie den Haar-Bereich des menschlichen Kopfes umgibt, und in die eine Reihe von Laserdioden derart eingesetzt ist, daß sie von der Innenfläche der Haube Licht in Richtung auf den Haar-Bereich emittieren, und
daß zur Unterstützung der chemischen Färbung die Wellenlänge und die Intensität der Laserdioden so gewählt sind, daß durch die Laserstrahlung die Struktur der α-Helices der einzelnen Haar-Makrofibrillen durch Einwirkung auf die Proteinstruktur der Haare durch eine Enzym-Koordinaten-Änderung bzw. eine Änderung der Redox-Potentiale effizient beeinflußt wird.

3. Vorrichtung nach Anspruch 2,
dadurch **gekennzeichnet**, daß die Laserdioden Licht im Bereich von etwa 780 nm emittieren.

4. Vorrichtung nach Anspruch 2 oder 3,
dadurch **gekennzeichnet**, daß zusätzliche Lichtquellen vorgesehen sind, die Licht über einen größeren Wellenlängenbereich emittieren.

5. Vorrichtung nach einem der Ansprüche 2 bis 4,
dadurch **gekennzeichnet**, daß in der Haube wenigstens eine Photodiode vorgesehen ist, die mit reflektiertem und/oder von wenigstens einer Laserdiode emittiertem Licht beaufschlagt wird, und deren Ausgangssignal an einer Steuereinheit für die Laserdioden zur Regelung der emittierten Lichtleistung angelegt ist.

6. Vorrichtung nach einem der Ansprüche 2 bis 5,
dadurch **gekennzeichnet**, daß die Haube an einem Ständer befestigt ist, und daß an der Innenfläche der Haube federartige Abstandshalter vorgesehen sind, die einen Mindestabstand zwischen Innenfläche der Haube und Kopf sicherstellen.

## Claims

1. Method of cosmetic hair treatment, particularly for the treatment of hair on the human head, wherein the hair is exposed to light and a wave length between roughly 600 and 1200 nm,
**characterised in that** at least one laser diode is used to assist the chemical dye, which has a wavelength and an intensity selected such that the structure of the α-helices of the individual macro-fibrils of the hair are influenced by the laser radiation on account of action produced by a change of the enzyme co-ordinates or a change of the redox potentials on the protein structure of the hair.

2. Apparatus for cosmetic hair treatment, particularly for the treatment of hair on the human head, comprising a light source irradiating the hair by means of light at a wavelength between roughly 600 and 1200 nm,
**characterised in that** a hood is provided which has an internal contour configured to surround the hair zone on the human head, wherein a number of laser diodes is so arranged that they emit light from the inner surface of the hood towards the hair Zone, and
that the wavelength and the intensity of the laser diodes are selected to assist the chemical dye in such a way that that the structure of the α-helices of the individual macro-fibrils of the hair are efficiently influenced by the laser radiation on account of action produced by a change of the enzyme coordinates or a change of the redox potentials on the protein structure of the hair.

3. Apparatus according to Claim 2,
**characterised in that** said laser diodes emit light at a range of 780 nm approximately.

4. Apparatus according to Claim 2 or 3,
**characterised in that** additional light sources are provided for the emission of light over a wider range of wavelengths.

5. Apparatus according to any of the Claims 2 to 4,
**characterised in that** at least one photodiode is provided in said hood, which is subjected to reflected light and/or light from at least one laser diode, and which outputs a signal applied to a controller for said laser diodes so as to control the emitted light flux.

6. Apparatus according to any of the Claims 2 to 5,
**characterised in that** said hood is fixed at a post, and that on the inner surface of said hood spring-like spacers are disposed to ensure a minimum spacing between the inner surface of the hood and the head.

## Revendications

1. Procédé de traitement cosmétique des cheveux, en particulier de la chevelure humaine, dans lequel les cheveux sont exposés à la lumière et à une longueur d'onde entre 600 et 1200 nm environ,
**caractérisé en ce** qu'au moins une diode laser est utilisée à l'appui de la coloration chimique, qui a une longueur d'onde et une intensité choisies de façon que la structure des hélices α des macro-fibrilles des cheveux individuelles soit influée par les rayons laser en vertu d'une action produite par un changement des coordonnées des enzymes ou respectivement par un changement des potentiels normaux d'oxydo-réduction sur la structure protéique des cheveux.

2. Appareil de traitement cosmétique des cheveux, en particulier de la chevelure humaine, comprenant une source lumineuse qui irradie les cheveux moyennant une lumière à une longueur d'onde entre 600 et 1200 nm environ,
**caractérisé en ce** qu'un bonnet est disposé dont le contour intérieur est configuré de façon qu'il entoure la zone de la chevelure sur une tête humaine, et dans lequel un nombre de diodes laser est monté de façon qu'elles émettent de la lumière de la surface intérieure du bonnet en direction vers la zone de la chevelure, et
en ce qu'à l'appui de la coloration chimique la longueur d'onde et l'intensité de la lumière sont choisies de façon que la structure des hélices α des macro-fibrilles des cheveux individuelles soit influée efficacement par les rayons laser en vertu d'une action produite par un changement des coordonnées des enzymes ou respectivement par un changement des potentiels normaux d'oxydo-réduction sur la structure protéique des cheveux.

3. Appareil selon la revendication 2,
**caractérisé en ce** que lesdites diodes laser émettent de la lumière dans une plage de 780 nm environ.

4. Appareil selon la revendication 2 ou 3,
**caractérisé en ce** que des sources lumineuses supplémentaires sont prévues pour l'émission de la lumière sur une plus large plage de longueurs d'onde.

5. Appareil selon une quelconque des revendications 2 à 4,
**caractérisé en ce** que dans ledit bonnet au moins une photodiode est prévue à laquelle on applique de la lumière réfléchie et/ou d'au moins une diode laser, et dont le signal de sortie est appliqué à une unité de commande pour lesdites diodes laser afin de régler le flux lumineux émis.

6. Appareil selon une quelconque des revendications 2 à 5,
**caractérisé en ce** que ledit bonnet est fixé à un poteau, et en ce qu'à la face intérieure dudit bonnet des écarteurs du type ressort sont disposés à assurer une distance minimum entre la surface intérieure du bonnet et la tête.
